Europäisches Patentamt

European Patent Office

Office européen des brevets

(11) Publication number: **0 115 029**
**A1**

(12) # EUROPEAN PATENT APPLICATION

(21) Application number: **83112829.3**

(22) Date of filing: **20.12.83**

(51) Int. Cl.³: **A 61 F 13/06**
**A 61 F 5/01**

(30) Priority: **31.12.82 JP 199380/82**
**18.08.83 JP 129216/82**

(43) Date of publication of application:
**08.08.84 Bulletin 84/32**

(84) Designated Contracting States:
**BE DE FR GB IT NL SE**

(71) Applicant: **Nakamura, Toshiro**
**Ha 132, Omori-cho**
**Oda-shi Shimane(JP)**

(72) Inventor: **Nakamura, Toshiro**
**Ha 132, Omori-cho**
**Oda-shi Shimane(JP)**

(74) Representative: **Dipl.-Ing. H. Hauck Dipl.-Phys. W.**
**Schmitz Dipl.-Ing. E. Graalfs Dipl.-Ing. W. Wehnert**
**Dr.-Ing. W. Döring**
**Neuer Wall 41**
**D-2000 Hamburg 36(DE)**

(54) Patella brace.

(57) A patella brace (8) comprising a securing member (11) for fixing the unsettled patella and the belt parts (12) for mutual connection purpose extending from both ends of the securing member to be applied by securing it around the knee articulation for protecting and redressing the affected part and removing the pains.

FIG.1

31 924

## SPECIFICATION

December 16, 1983

## Title of the Invention

### Patella Brace

## Background of the Invention

The present invention relates to a patella brace as a therapeutic gadget to be applied to the patent's body for protecting and redressing unsettled patella and removing pains of the patella and thighbone troubles or more particularly the patella trouble.

A patella/thighbone trouble needs to be remedied for depending upon the patient's individual condition, that is to say, the kind and degree of the trouble. As for the patients of serious trouble requiring an operation, thoroughgoing studies have been achieved and therapies by means of an operation are widely resorted to. On the other hand, however, those having a rather slight trouble are not remedied for sufficiently, with studies in application of therapeutic gadget not being pursued with serious concern.

This is so said because the conventional patella braces have many shortcomings. Restricting the patient's physical movements very much, these gadgets not only obstructs the patient's daily life a great deal but also affect functional troubles in other parts of the body. Especially in the case of women, these gadgets are apt to be abhored for putting on for reason of their unattractive appearance.

- 1 -

Problems are that there are considerable numbers of girls among the sport club members of junior and senior high schools (especially of volleyball and basketball club members) who grieve over their patella trouble. Their symptoms are in most cases that the patella moves laterally outward (that is to the gastrocnemius side). But their causes and the reasons why the troubles concentrates on girls are not known, and consequently there isn't any decidedly effective fundamental therapy so far. The only remedy available seems to be in redressing said trouble by putting on a therapeutical gadget. Such gadget are, however, generally not handy to handle and are very bulky, causing repulsive feel to the patients besides being unattractive in appearance.

The knee joint is the articulation which is situated at the lower end of thighbone, on the top of the shinbone and behind the patella, where these bones are jointed by many ligaments such as windgall, inside and outsidee accessory ligaments and cruciate ligaments of the knee, which limit at the same time the direction and the scope of the body movement.

The reinforcement by these muscles made to the articulation being very powerful, which is reckonable by the fact that a fracture of bone is rather more often taking place than a dislocation of joint by an external

- 2 -

injury, that the balance in reinforcement strength is badly destroyed affecting outright to other parts of the body when the trouble takes place. The most liable part to be affected is the patella.

In addition, especially when the therapy is applied to cure subluxation of patella or the chondranacia patellae by putting on the gadget, all bone and muscles are moved or varied in positions in a complicated manner as the knee is expanded and/or contracted, and this makes the therapy almost impossible by mere forceful pulling, meaning that it is impossible to secure the part in immovable state is very difficult.

With these things in view, most of the conventional patella brace are of the type that aim to keep the patella immovable by enclosing it. All others having an opening as large as the size of patella are of cylindrical form made of elastic cloth, like the knee supporter for sport use, which fixes the patella immovable on a fixative arm mostly made of rubber. This type of cylindrical form gadget is difficult to drop off after being put on as its feature, making it certainly suitable for the sports requiring vilent. movements. For putting on this gadget, however, it is necessary to bend the body deeply, which makes it very inconvenient for the people having trouble at the patella, even to the patients whose trouble is light in degrees, not

- 3 -

requiring any help from other person. In the case of cylindrical brace, the supporting pressure to be applied to patella is difficult to be reduced gradually in accordance with the progress of therapy. Since it is fastening the part including well above and below the patella, its shortcomings are found in that it restricts. the free movement of body to a large measure.

Summary of the Invention.

The fist objective of this invention is to offer a patella brace provided with a high curing effect which is light in weight and inexpensive in cost while reducing the repulsive and unfortable feel when put on.

The present invention has for its additional objective to supply a patella brace which can be put on easily even by a patient deprived of a sufficient physical movement function without any help by a third person.

Brief Description of the Drawings

Fig. 1 is a perspective view showing an embodiment of a patella brace of the present invention. Fig. 2 is a front view of the patella brace shown in Fig. 1 when it is put on. Fig. 3 is a perspective view showing said brace which uses a separate belt for the connection.
Fig. 4-(a) is a front view and Fig. 4-(b) is its side view respectively, both showing the method for putting it on. Fig. 5 is a perspective view of said brace provided with an

- 4 -

anti-slide member .

Detailed Desctiption of the Invention

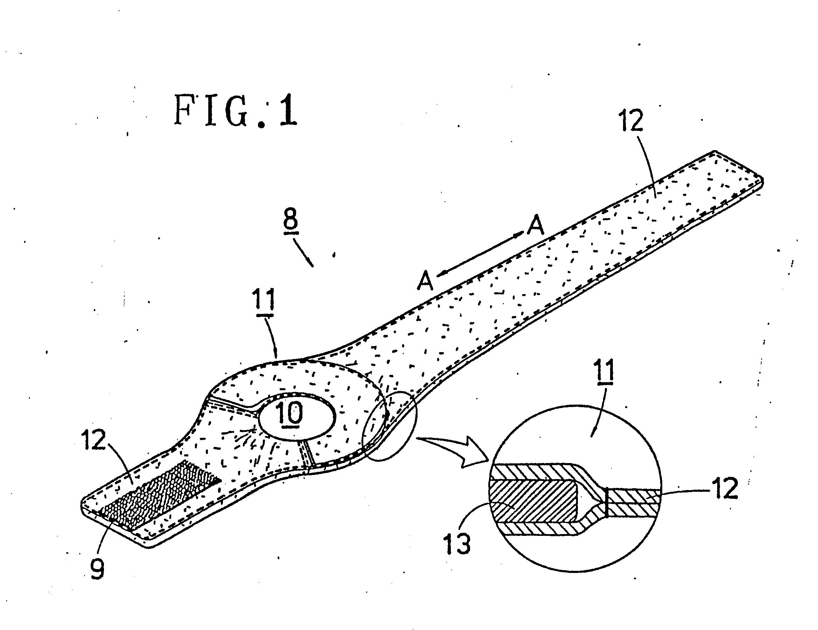

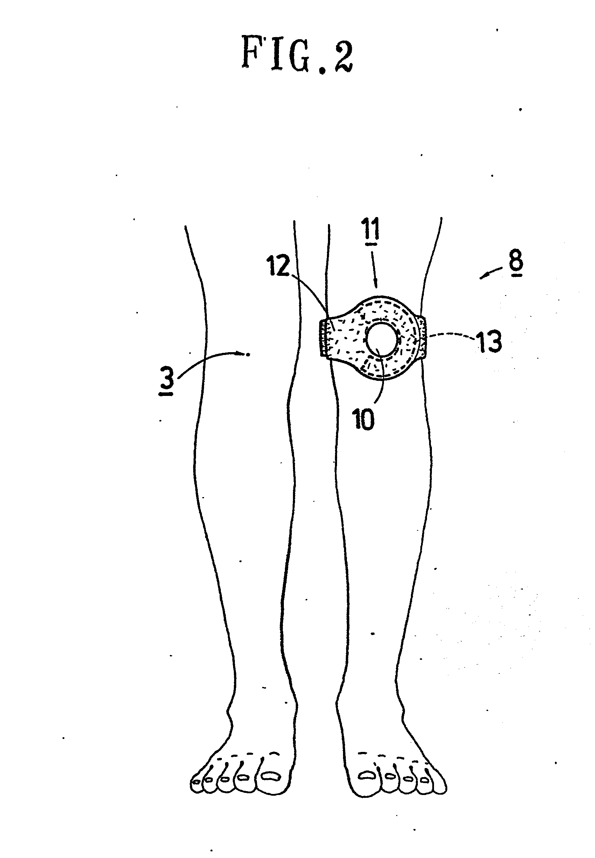

Both Fig. 1 and Fig. 2 show one of the embodiments of the present invention. Fig. 1 is a perspective view of the patella brace (8), including a magnified cross-sectional view of a portion thereof. Fig. 2 is a front view around the knee-joint of a patient having put on the patella brace (8). As evident from the drawings, the patella brace (8) in accordance with the present invention comprises a securing member (11), which has an opening (10) which it surrounds and two belt parts (12) (12) which extend from both ends of the securing member (11). Said patella brace (8) is made so that it can be connected at each of its ends, the method of connection thereof being not limited to the plane fastener, which is the case adopted herein.

The putting on of this patella brace is much easier when compared with the conventional cylindrical type supporters. Positioning the opening (10) of the patella brace. at the patella, the belt parts (12) (12) are put around the joint and are fastened with the plane fastener, which is all that is required to do for putting on. Since it is this much of the structure and form, the securing member is very small and the entire unit is light in weight, causing but little feel of foreign matter and restricting very little of free movement of the patient body, which allow a long time application without any impediment, that means ideal as a gadget.

The method of putting it on being so easy in mere connecting each other of the belt parts (12) (12), said belt parts can be made of cloth or the like, preferably having some elasticity to the direction of A—A in Fig. 1, it is assumed.

The form, of the opening (10) is preferred to be determined in accordance with the form and size of the patient's patella, which it would be considered sufficient with a several different patterns to meet the requirements of a quite many patients.

The securing member (11) comprises a fixture (13) having some resilience and thickness in all or some part of its body, which surrounds the opening part (10). In an instance of this embodiment, the fixture (13) is made of silicon rubber, with 2 sheets of cloth, holding the fixture (13) in between as the securing member (11). The fixture (13) is given a C letter form in the instance of this embodiment. In any case, a fixture is serving its purpose as far as it supports the patella firmly. It is therefore not limited to the present form. Either a doughnut form or a U letter form will do . And any material as far as it is fixative and resilient will be all right, regardless whether it is made of natural rubber, urethane form or any other material.

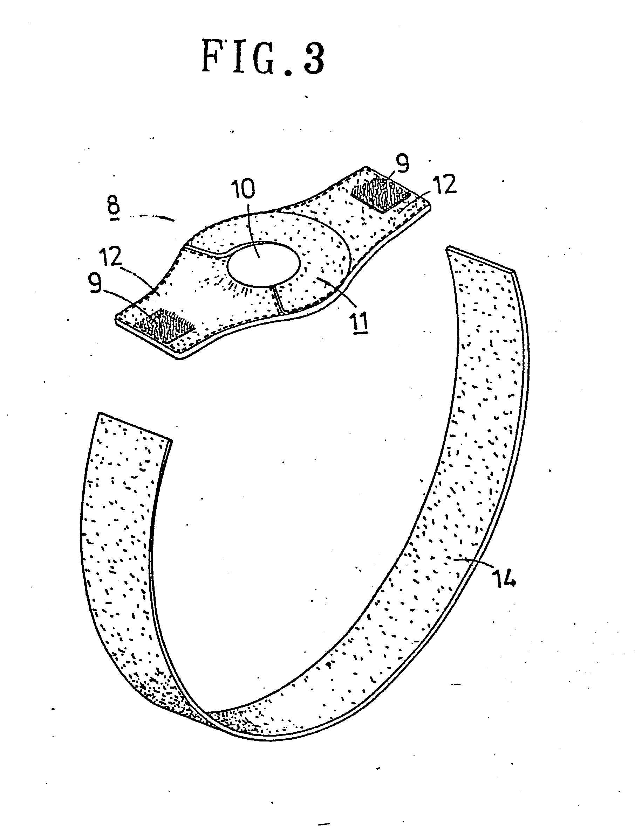

As examples of mutual connection method of belt parts (12) ● (12) other than the plane fastner, a separate connecting belt (14) can be conceived, with the belt parts (12)●(12) being made small (refer to Fig. 3). In such case the patella brace (8) can be smaller by the extent that the belt parts (12)●(12) become smaller.

As for the application of the patella brace (8) in accordance with the present invention on the patient's physique, it is very easy as already mentioned in the above to put it on and off.

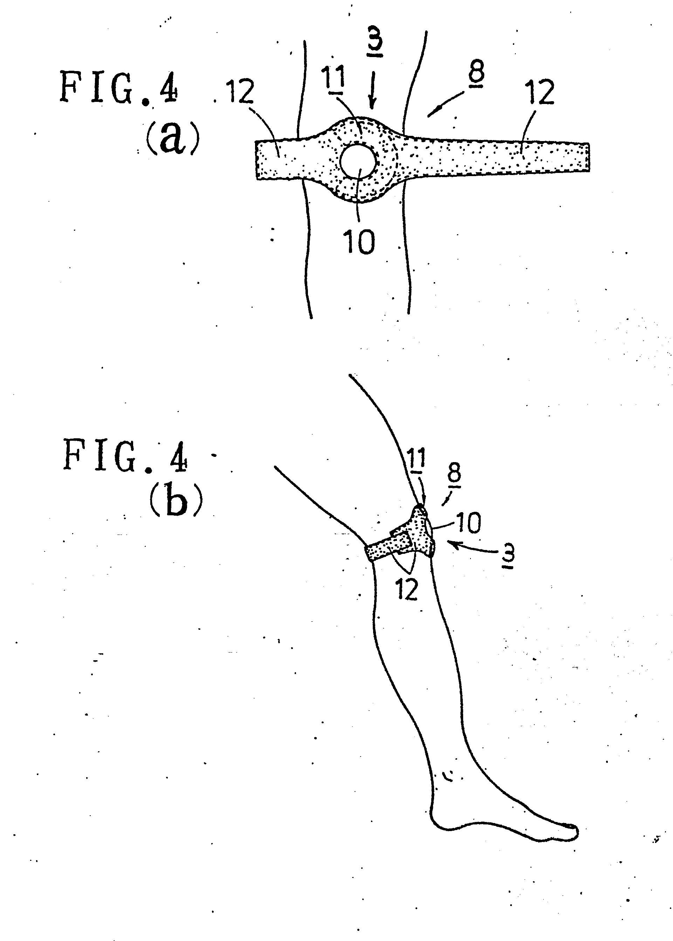

As it is evident from the illustration given in Fig. 4-(a) and -(b), it is only to be done by applying the opening part (10) of the patella brace (8) onto the patella see Fig. 4-(a) , getting round the belt parts to the back of patella and fastening the belts at an appropriate position so that the pressing force will be applied to the affected part in accordance with extent of the trouble, as seen in Fig. 4-(b). With the part restricting the movement of knee being small, when it is expanded and/or contracted, the putting it on is very easy for any patient.

In any case of the unsettled parella trouble that are subject to therapy by this sort of therapeutical gadget, the unsettled patella moves generally to the lateral direction when the patient stands up straight.

The patella (3) being apt to swing to the right in Figs. 4-(a) and -(b), and if the present patella brace (8) is used to cure such trouble, apply the opening part (10) onto the patella and keep the fixture to position at the right side of patella, shifting the entire unit of gadget to the left side so that an appropriate pressure is applied to the patella. For this, it is very easy to do by pulling both belt part ends (12)•(12) , which are extended from the securing member (11) with both hands and connecting both ends (12)•(12) around the patella (here in this example, with the plane fastner). This connection is of course done for applying pressure to patella and also for securing the brace, which has an effect at the same time to prevent the securing member (11) (at the opposite side of the fastener when connected each other) from floating up.

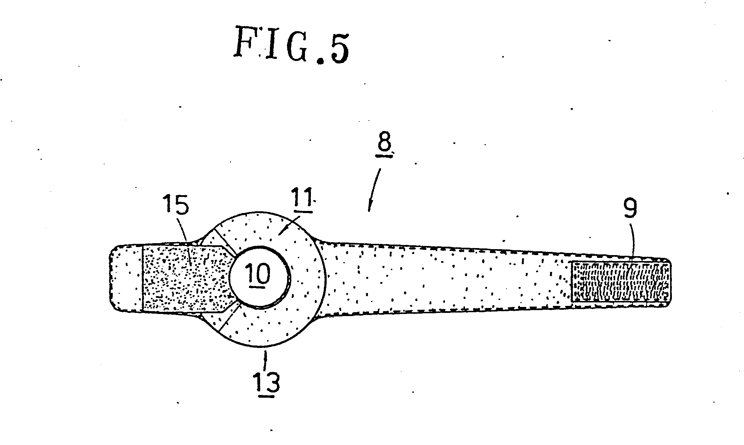

Fig. 5 shows an example in which the brace has on its back side an anti-slide member (15), of which material is composed of two-layer construction of nap-raised surface of synthetic rubber sheet and the cloth which is pasted onto the main body to compose the patella brace (3). By providing this anti-slide member (15), other part of the back side excepting the anti-slide member (15) can be of the material which is easy to slide, making it possible to prevent the gadget from sliding away to the unwanted direction, when the brace is in application. Furthermore, by providing an anti-slide member (15) on the back side and slippable material on the surface, it will help avoid the gadget to shift lessening the friction with the garment which also helps to prevent the garment from wearing out of shape. This makes the present brace excellent in feeling when put on.

In the instance of the present embodiment, the material used for the anti-slide member (15) is made of two-layer construction of synthetic rubber and cloth. This part may come in a thin rubber sheet and may be sewn up instead of being pasted. The aim is to make it feel not crusty when the knee is expanded and/or contracted and not to kill the anti-sliding effect by making ripples and consequently friction. Even other materials than the two-layer construction or thin rubber sheet will do for the material to be used, meaning that it is not limited to these construction and the material above mentioned.

If the patella brace is put on in a manner in which the anti-slide member (15) comes to the inside of the knee, the divergence of the fixed position will be less than otherwise and the feeling of putting it on is more agreeable, it has been known through experiments. It can be endorsed also from an anatomical fact that the fitting of the gadget is easier in that state because there is a plateau like projection at the end of thighbone on the

It is to be noticed that the position for pasting or sewing the anti-slide member (15) will do anywhere on the backside of the main body, which is not limiting the present invention

Thus the patella brace in accordance with the present invention can be easily put on and off, with a very excellent feel in applying being assured.

What is claimed is:

1.     A patella brace used as a therapeutic gadget to be applied on physique for curing the patella trouble in protecting and redressing unsettled patella and for removing pains characterized in that it comprises a securing member, which fixes the unsettled patella, and an opening part, which exposes said patella, wherein said securing member contains a soft resilient material and at both ends thereof each belt part extends for connecting with each other.

2.     A patella brace in accordance with claim 1, wherein at least one of said two extending belt parts as the mutual connection means has a plane fastner fixed.

3.     A patella brace in accordance with claim 1 or claim 2, wherein at least at one part of the back surface of the main body an anti-slide member is provided.

4.     A patella brace in accordance with claim 3, wherein said anti-slide member is made of natural or synthetic rubber sheet which is fixed either by pasting or sewing on the back surface of the main unit.

FIG.1

# FIG.2

# FIG.3

FIG.4
(a)

FIG.4
(b)

# FIG.5

0115029

Application number

EP  83 11 2829

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | EP-A-0 010 389  (PALUMBO)<br>* Claims 1, 2; figures 1-4 * | 1 | A 61 F   13/06<br>A 61 F    5/01 |
| Y | US-A-4 084 584  (DETTY)<br>* Claims 1-3; figures 1, 4 * | 1 | |
| Y | DE-U-8 115 670  (HANE)<br>* Claims 1, 5; figures 1-3 * | 1 | |
| A | US-A-4 287 885   (APPLEGATE) | | |
| A | US-A-3 804 084   (LEHMAN) | | |
| A | US-A-3 318 305   (SCHULTZ) | | TECHNICAL FIELDS SEARCHED (Int. Cl. ³) |
| A | FR-A-  840 438   (MANDL et al.) | | A 61 F    5/00<br>A 61 F   13/00 |
| P,A | US-A-4 370 978   (PALUMBO) | | |

The present search report has been drawn up for all claims

| Place of search<br>BERLIN | Date of completion of the search<br>28-03-1984 | Examiner<br>KANAL P  K |
|---|---|---|

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03.82